# FASCICULE DE BREVET EUROPEEN

(11) **EP 0 692 236 B1**
(45) Date de publication et mention de la délivrance du brevet: **10.07.1996**
(21) Numéro de dépôt: 95401083.1
(22) Date de dépôt: 10.05.1995
(51) Int. Cl.: A61K 7/00, A61K 7/50, A61K 7/02

(54) **Composition de nettoyage contenant des grains lipides**
Reinigendes Mittel enthaltend lipidische Körner
Cleaning composition containing lipidic grains

(30) Priorité: 17.06.1994 FR 9407481
(43) Date de publication de la demande: 17.01.1996
(73) Titulaire: L'OREAL, F-75008 Paris (FR)
(72) Inventeur: Khayat, Carine, F-94210 La Varenne (FR); Candau, Didier, F-91570 Bievres (FR)
(74) Mandataire: Lhoste, Catherine

(56) Documents cités:
- EP-A- 0 376 852
- EP-A- 0 412 865
- EP-A- 0 452 202
- EP-A- 0 506 197

## Description

La présente invention a pour objet une composition de nettoyage de la peau sous forme d'émulsion huile-dans-eau (H/E), pouvant être utilisée notamment pour le nettoyage de la peau du visage, du cou et/ou du corps humain. En particulier, cette composition, qui permet une élimination en douceur des cellules mortes de la peau, constitue un produit "3 en 1", particulièrement appropriée aux peaux sensibles.

L'invention se rapporte aussi à un procédé pour nettoyer la peau du corps et/ou du visage.

Les produits de gommage ou exfoliants, encore appelés "scrubs", contiennent des particules exfoliantes qui sont constituées de matières abrasives telles que la poudre de polyéthylène, la poudre de coques de noix ou la poudre d'amande d'abricot. Malheureusement, ces produits de gommage présentent l'inconvénient d'être irritants du fait même de la présence de ces particules qui frottent sur la peau sans fondre et subsistent à sa surface. En raison de leur caractère irritant, il n'est pas possible d'utiliser quotidiennement ces produits de gommage ; selon la sensibilité de la peau, l'utilisation doit en être plus ou moins espacée, notamment de plusieurs jours, voire plusieurs semaines pour les peaux très sensibles.

Par ailleurs, pour remédier à cette irritation, il est nécessaire, après usage, d'appliquer une crème de soin apportant à la peau protection et hydratation.

Il subsiste donc le besoin d'un produit gommant confortable, qui ne comporte pas de particules exfoliantes abrasives et qui ne nécessite pas ultérieurement l'application d'une crème de soin.

La composition selon l'invention permet justement de remédier aux inconvénients décrits ci-dessus.

En effet, de façon surprenante, la demanderesse a trouvé qu'il était possible d'avoir un produit de gommage "3 en 1", c'est-à-dire un produit qui assure à la fois le nettoyage, le gommage et le soin de la peau, ne contenant pas de particules abrasives, en utilisant une émulsion huile-dans-eau comprenant des grains lipidiques chargés d'un actif nettoyant.

Ainsi, l'invention a pour objet une composition de nettoyage de la peau sous forme d'émulsion huile-dans-eau, caractérisée en ce qu'elle comprend un émulsionnant ayant une balance hydrophile-lipophile inférieure ou égale à 12 et des grains lipidiques solides non hydrophiles ayant un point de fusion inférieur à 50°C, ces grains incluant un actif nettoyant.

Certes, il est connu par le document FR-A-2 649 608 d'utiliser, dans un gel aqueux, des grains constitués d'une substance lipidique solide non hydrophile ayant un point de fusion inférieur à 50°C, pour rendre le gel plus confortable à l'application. Mais ce gel, même chargé de grains lipidiques, est moins confortable qu'une émulsion et ne peut pas apporter un effet soin aussi important que celui d'une émulsion du fait de son absence d'huile.

Contrairement à toute attente, il n'était pas évident d'incorporer ces grains lipidiques dans une émulsion. En effet, dans certaines conditions, l'incorporation de grains lipidiques dans une émulsion huile-dans-eau entraîne une fusion de la ou des substances lipidiques des grains avec la phase huileuse, si bien que le produit obtenu ne peut plus servir au gommage et/ou au nettoyage de la peau.

Cette fusion apparaît aussi dans une émulsion eau-dans-huile : la ou les substances lipidiques des grains fusionnent avec la phase continue et l'émulsion obtenue est instable.

La demanderesse a trouvé qu'en utilisant une émulsion huile-dans-eau et un émulsionnant ayant une balance hydrophile-lipophile (HLB) inférieure à 12, et plus particulièrement comprise entre 6 et 12, on pouvait incorporer les grains lipidiques dans une émulsion sans que les grains fusionnent avec la phase huileuse. Par contre, si l'on utilise des émulsionnants ayant une balance hydrophile-lipophile supérieure à 12 comme c'est couramment le cas pour la préparation d'émulsion huile-dans-eau, on constate que la ou les substances lipidiques des grains ont tendance à se solubiliser dans la phase huileuse de l'émulsion, comme le montrent les exemples comparatifs donnés ci-dessous.

L'invention a par ailleurs pour objet une composition démaquillante, caractérisée en ce qu'elle contient la composition définie ci-dessus et en ce que l'actif nettoyant est démaquillant.

L'invention a aussi pour objet un procédé pour nettoyer et/ou gommer la peau du corps et/ou du visage et/ou du cou, caractérisé en ce qu'il consiste à appliquer sur la peau la composition définie ci-dessus, à masser la peau avec la composition pour éliminer les cellules mortes et éventuellement à rincer la peau.

L'invention a encore pour objet l'utilisation de la composition définie précédemment, pour nettoyer et/ou gommer la peau du corps humain et/ou du visage et/ou du cou.

Le nettoyage du corps humain concerne aussi bien les bras que les jambes, le ventre et le dos.

La composition selon l'invention a une triple action. Elle a, en effet, une action de nettoyage, une action de gommage et une action de soin.

Le nettoyage de la peau est obtenu grâce à la présence des grains lipidiques qui contiennent un actif nettoyant qui va absorber les salissures et les impuretés de la peau. Par ailleurs, dans le cas du démaquillage, les grains, qui sont lipophiles, se fondent au maquillage pour mieux l'éliminer ensuite.

Le gommage de la peau est réalisé par le massage des grains sur la peau qui va entraîner l'élimination des cellules mortes ; les grains vont fondre au cours de cette action mécannique, sans provoquer d'irritation.

Le soin est obtenu grâce à la texture et au choix des constituants de l'émulsion : huiles et actifs.

Comme indiqué précédemment, l'émulsionnant utilisable dans la composition de l'invention doit avoir une balance hydrophile-lipophile inférieure à 12, et plus particulièrement comprise entre 6 et 12. On peut notamment utiliser comme émulsionnant le cétéarylglucoside vendu sous la dénomination Montanov 68 par la société Seppic, qui a une valeur de HLB de 10. On peut aussi utiliser des mélanges d'émulsionnants qui donnent au final une valeur de HLB allant de 6 à 12, les émulsionnants étant par exemple choisis parmi les émulsionnants suivants : stéarate ou tristéarate de sorbitan, stéarate de PEG-40, stéarate de PEG-50, stéarate de PEG-100, stéarate de glycéryle et polysorbate 60.

Dans la composition selon l'invention, il est particulièrement important que le point de fusion des grains lipidiques soit inférieur à 50°C, et de préférence aille de 30 à 45°C, de telle sorte que les grains fondent par simple application sur le corps, avec un léger frottement ou massage.

Parmi les subtances lipidiques solides non hydrophiles formant les grains lipidiques, on peut notamment mentionner les glycérides hémisynthétiques, certaines substances grasses naturelles ainsi que certains composés gras synthétiques.

Parmi les glycérides hémisynthétiques, on peut notamment utiliser les triglycérides d'acides gras saturés linéaires ayant de 8 à 18 atomes de carbone, à indice d'hydroxyle < 30 et indice d'iode < 3, et parmi ceux-ci, ceux vendus sous les dénominations Lipocire ou Suppocire par la société Gatefosse, et en particulier les Lipocire A ou DM, les Suppocire AlM, AM, BM, CM, DM, Al, A, B, C et D, et ceux vendus sous les dénominations Witepsol^{R} ou Softisan par la société Dynamit Nobel, et en particulier les Witepsol^{R} H₃₂, H₃₅, H₃₇, H₃₉ et H₄₂.

Parmi les substances naturelles, on peut citer les beurres ou fractions solides de corps gras végétaux tels que par exemple le beurre de karité, le beurre de cacao, l'huile de coprah et ses dérivés.

Parmi les composés gras synthétiques, on peut citer les cires de silicone telles que les produits vendus par la société Wacker sous la dénomination Cire silicone VP 1622 ou Silicone Copolymer F 755.

Les substances lipidiques énumérées ci-dessus peuvent être utilisées seules ou sous forme d'un mélange, et, dans ce dernier cas, il est préférable de parler de domaine ou de zone de fusion, plutôt que de point de fusion.

Il est également possible selon l'invention d'utiliser des substances lipidiques ayant un point de fusion supérieur à 50°C, mais, dans ce cas, il est nécessaire d'avoir recours à la présence d'un adjuvant lipidique de zone de fusion plus basse pour ajuster le point de fusion du mélange à une valeur inférieure à 50°C.

Parmi les subtances lipidiques à point de fusion supérieur à 50°C, on peut citer des triglycérides tels que le Softisan 154 vendu par la société Dynamit Nobel, les cires animales, végétales, minérales ou synthétiques ou leurs dérivés, et en particulier la cire d'abeille, le blanc de baleine, la cire de candellila et ses dérivés hydrogénés, la cire de carnauba et ses dérivés hydrogénés, les paraffines, les ozokérites et, le produit commercialisé sous le nom Elfacos C₂₆ par la société Akzo Chemie.

Parmi les adjuvants lipidiques destinés à abaisser le point de fusion des substances lipidiques fondant au-dessus de 50°C, on peut notamment mentionner les alcools gras saturés linéaires en C₁₄ et C₁₆, les alcools ramifiés en C₁₂ à C₂₄, les esters gras, les acides gras insaturés, les mélanges complexes de lipides tels que les huiles végétales, ou les huiles de silicone.

En outre, il est possible, pour ajuster la consistance ou la viscosité des grains lipidiques, d'introduire dans le mélange, des argiles modifiées ou leur dispersion huileuse, des silices, des savons métalliques ou tout autre structurant des corps gras. On peut en particulier, pour ajuster la dureté des grains, introduire du monodiisostéarate de glycéryle.

L'actif nettoyant contenu dans les grains de la composition de l'invention peut être choisi parmi les farines de céréales, les argiles et les huiles démaquillantes. Selon une forme de réalisation préférée de l'invention, l'agent nettoyant est une farine de céréale.

Il est connu d'utiliser des farines pour nettoyer la peau (voir à cet effet le document FR-A- 2 499 097). Cependant, leur introduction dans une émulsion cosmétique pose différents problèmes : du fait de la présence d'eau dans l'émulsion, les farines vont, d'une part, développer des bactéries, dont le développement est difficilement contrôlable et entraîne une pollution de l'émulsion, et, d'autre part, elles vont se gonfler en absorbant l'eau et donner un empois qui rend désagréable l'application de l'émulsion. Le fait d'introduire les farines dans les grains lipidiques conformément à l'invention permet de protéger la farine de la phase aqueuse et donc d'éviter les problèmes mentionnés ci-dessus tout en conservant les propriétés de nettoyage des farines.

Comme farines de céréale utilisables dans la composition de l'invention, on peut notamment citer la farine de froment, la farine de blé, la farine d'avoine et la farine de soja.

Comme argiles utilisables dans la composition de l'invention, on peut notamment citer le kaolin.

Comme huiles démaquillantes utilisables dans la composition de l'invention, on peut citer notamment le palmitate d'éthyl-2 hexyle, l'adipate de diéthyl-2 hexyle, l'adipate de dioctyle, l'adipate de di-isopropyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle.

Les grains lipidiques peuvent également contenir des adjuvants choisis parmi les actifs hydrophiles ou lipophiles, les parfums, les conservateurs, les matières colorantes, notamment les pigments, et les agents antioxydants.

Les actifs lipophiles ou hydrophiles peuvent être notamment des actifs hydratants tels les polyols ; des céramides ; des agents apaisants tels que le thé vert. Il est tout particulièrement intéressant d'incorporer dans les grains lipidiques des actifs thermosensibles, tels que les vitamines ; en effet, ces actifs ne peuvent être introduits directement dans l'émulsion sans subir de préjudice du fait que l'émulsion est préparée à chaud et que ces actifs se dégradent facilement à la chaleur, alors que leur introduction dans les grains lipidiques est réalisée à la température de fusion de la ou des substances lipidiques, c'est-à-dire à une température inférieure à 50°C.

Le diamètre moyen des grains lipidiques peut être compris dans de très larges limites, par exemple de 50 à 10.000 µm. Il est, néanmoins, de préférence compris dans la gamme allant de 50 à 2.000 µm, et plus particulièrement de 630 à 1200 µm.

Selon une forme de réalisation préférée de l'invention, les grains lipidiques sont obtenus par cryobroyage. Le procédé de préparation consiste à faire fondre la ou les substances lipidiques au bain marie à une température de 2-3°C supérieure à la zone de fusion des substances lipidiques à disperser dans le produit fondu, à l'aide d'une hélice, les adjuvants, puis, après obtention d'un mélange homogène, à enlever le bain marie et à laisser refroidir le mélange à la température ambiante sous agitation. Peu avant la solidification du mélange, on retire l'hélice et on soumet le solide obtenu à une congélation brutale à une température inférieure à 0°C, et de préférence comprise entre -80°C et -120°C, à l'aide de l'azote liquide. On effectue ensuite un broyage cryogénique jusqu'à obtention d'une granulométrie comprise entre quelques µm et quelques mm. Ce procédé permet d'introduire les actifs thermosensibles dans les grains lipidiques sans les chauffer, et donc sans les dégrader.

Les grains lipidiques utilisables selon l'invention peuvent contenir de 75 % à 100 % en poids, et de préférence de 85 % à 95 % en poids de substances lipidiques, de 0,5 % à 10 % en poids, et de préférence de 1 % à 5 % en poids d'actifs, et de 0,1 % à 5 % en poids, et de préférence de 0,1 % à 2 % en poids d'autres adjuvants. S'ils contiennent un composé pour ajuster la dureté des grains, notamment le monodiisostéarate de glycéryle, ce dernier peut être présent en une quantité allant de 0,01 % à 20 % en poids, et de préférence de 0,01 % à 15 % en poids.

La composition selon l'invention peut en outre contenir de 0,5 % à 10 % en poids, et de préférence de 0,5 % à 5 % en poids de grains lipidiques par rapport au poids total de la composition.

Dans la composition selon l'invention, la phase huileuse peut représenter de 10 % à 40 % en poids par rapport au poids total de la composition.

Comme huiles utilisables dans l'invention, on peut citer les huiles végétales (huile d'abricot), les huiles d'origine animale, les huiles minérales (huile de vaseline), les huiles de synthèse, les huiles siliconées et/ou les huiles fluorées. La phase huileuse peut contenir aussi d'autres corps gras tels que les acides gras, les alcools gras (alcool cétylique) et les cires.

De façon connue, la composition selon l'invention peut de plus contenir des additifs habituels dans le domaine cosmétique, tels que des actifs hydrophiles ou lipophiles, des gélifiants hydrophiles ou lipophiles, des conservateurs, des antioxydants, des parfums, des charges, des filtres, des matières colorantes et encore des vésicules lipidiques.

On peut citer notamment comme actifs les polyols tels que la glycérine, les hydrolysats de protéine et les céramides. Ces adjuvants, selon leur nature, sont utilisés dans les proportions habituelles des compositions cosmétiques, et, par exemple, de 0,01 % à 10 % en poids par rapport au poids total de la composition, et ils sont, selon leur affinité, introduits dans la phase aqueuse ou dans la phase huileuse de l'émulsion.

Les exemples qui suivent sont donnés à titre illustratif afin de mieux faire comprendre l'invention. Les quantités indiquées sont des pourcentages en poids.

### EXEMPLE 1 : Nettoyant doux

### *Composition des grains lipidiques :

| | |
|---|---|
| Lipocire DM (vendu par la société Gattefosse) | 88 % |
| Monodiisostéarate de glycéryle | 5 % |
| Farine de froment | 5 % |
| Pigments | 2 % |

### *Composition cosmétique :

| *Phase huileuse :* | |
|---|---|
| Cétéarylglucoside (Montanov 68 de la société Seppic) (émulsionnant) (HLB=10) | 4,25 % |
| Alcool cétylique | 0,75 % |
| Huile d'abricot | 25,00 % |

| *Phase aqueuse :* | |
|---|---|
| Gomme de xanthane (gélifiant) | 0,20 % |
| Eau | 64,80 % |
| *Grains lipidiques* | 5 % |

### Mode de préparation de l'émulsion :

On chauffe le cétéarylglucoside et l'alcool cétylique à 80°C et, parallélement, on chauffe l'huile végétale à 60°C ; puis, on les mélange de manière homogène. On prépare par ailleurs la phase aqueuse en mélangeant la gomme de xanthane et l'eau à 80°C sous agitation. Ensuite, on verse lentement la phase huileuse dans la phase aqueuse en agitant fortement. On laisse refroidir le mélange jusqu'à température ambiante. On y ajoute ensuite les grains lipidiques obtenus par cryobroyage.

Le nettoyant doux obtenu a l'aspect d'une crème dans laquelle on voit les grains lipidiques colorés. Ces grains conservent bien leur couleur au cours du temps et l'émulsion reste bien blanche.

Le produit obtenu a été testé sur un panel de 13 personnes expertes dans le domaine cosmétique qui l'ont appliqué sur le visage tous les soirs pendant une semaine. Le produit a été jugé extrèmement novateur de par sa texture, très doux à l'application et très efficace pour le nettoyage de la peau. Après application et rinçage, la peau de ces personnes était douce, nourrie et hydratée. Malgré son efficacité comme exfoliant, toutes les personnes ont continué à l'utiliser quotidiennement sans problème.

### EXEMPLE COMPARATIF 1 : Nettoyant

### *Composition des grains lipidiques :

| | |
|---|---|
| Lipocire DM (vendu par la société Gattefosse) | 88 % |
| Monodiisostéarate de glycéryle | 5 % |
| Farine de froment | 5 % |
| Pigments | 2 % |

### *Composition cosmétique :

| *Phase huileuse :* | |
|---|---|
| Stéarate de sucrose (vendu sous la dénomination Crodesta F 160 par la société Croda) (émulsionnant) | 3,00 % |
| Stéarate de sucrose/distéarate de sucrose (mélange vendu sous la dénomination Crodesta F110 par la société Croda) (émulsionnant) | 2,00 % |
| Alcool cétylique | 0,75 % |
| Huile d'abricot | 25,00 % |

| *Phase aqueuse :* | |
|---|---|
| Gomme de xanthane (gélifiant) | 0,20 % |
| Eau | 64,05 % |
| *Grains lipidiques* | 5 % |

Le procédé de préparation est le même que dans l'exemple 1. La valeur HLB du mélange d'émulsionnants utilisés est de 13,5.

Dans le produit obtenu, I'émulsion prend la couleur des grains du fait de la diffusion des pigments dans l'émulsion et les grains se décolorent complètement au cours du temps.

### EXEMPLE 2 : Nettoyant doux

### *Composition des grains lipidiques :

| | |
|---|---|
| Lipocire DM (vendu par la société Gattefosse) | 88 % |
| Monodiisostéarate de glycéryle | 5 % |
| Farine d'avoine | 5 % |
| Pigments | 2 % |

### *Composition cosmétique :

| *Phase huileuse :* | |
|---|---|
| Tristéarate de sorbitan (émulsionnant) | 0,9 % |
| Stéarate de PEG-40 (émulsionnant) | 2,0 % |
| Alcool cétylique | 2,5 % |
| Huile d'abricot | 13,5 % |

| *Phase aqueuse :* | |
|---|---|
| Glycérine | 3,0 % |
| Hydrolysat de protéine de blé | 0,5 % |
| Eau | 72,6 % |
| *Grains lipidiques* | 5 % |

Le procédé de préparation est le même que dans l'exemple 1. La valeur HLB du mélange d'émulsionnants utilisé est de 12,3. On obtient une crème qui nettoie bien et laisse une peau douce.

### EXEMPLE COMPARATIF 2 : Nettoyant doux

### *Composition des grains lipidiques :

| | |
|---|---|
| Lipocire DM (vendu par la société Gattefosse) | 88 % |
| Monodiisostéarate de glycéryle | 5 % |
| Farine d'avoine | 5 % |
| Pigments | 2 % |

### *Composition cosmétique :

| *Phase huileuse :* | |
|---|---|
| Stéarate de sucrose (vendu sous la dénomination Grilloten PSE 141G par la société Rita) (émulsionnant) (HLB=14,9) | 2,9 % |
| Alcool cétylique | 2,5 % |
| Huile d'abricot | 13,5 % |

| *Phase aqueuse :* | |
|---|---|
| Glycérine | 3,0 % |
| Hydrolysat de protéine de blé | 0,5 % |
| Eau | 72,6 % |
| *Grains lipidiques* | 5 % |

Le procédé de préparation est le même que dans l'exemple 1. La valeur HLB de l'émulsionnant utilisé est de 14,9. Au cours de l'étude de stabilité de la composition obtenue à différentes températures, on observe une coloration de plus en plus importante de la composition tandis que les grains deviennent de plus en plus mous et décolorés.

### EXEMPLE 3 : Crème démaquillante

### *Composition des grains lipidiques :

| | |
|---|---|
| Lipocire CM (vendu par la société Gattefosse) | 88 % |
| Palmitate d'éthyl-2 hexyle | 10 % |
| Pigments | 2 % |

### *Composition cosmétique :

| *Phase huileuse :* | |
|---|---|
| Stéarate de glycéryle (émulsionnant) | 3 % |
| Stéarate de PEG-50 (émulsionnant) | 3 % |
| Alcool cétylique | 5 % |
| Huile de vaseline | 12 % |
| Huile de silicone volatile | 12 % |

| *Phase aqueuse :* | |
|---|---|
| Glycérine | 3 % |
| Eau | qsp 95 % |
| *Grains lipidiques* | 5 % |

Le procédé de préparation est le même que dans l'exemple 1. On obtient une crème qui nettoie bien, laisse une peau douce et démaquille parfaitement grâce à la présence d'huile démaquillante.

### EXEMPLE 4 : Lait démaquillant

### *Composition des grains lipidiques :

| | |
|---|---|
| Lipocire DM (vendu par la société Gattefosse) | 92 % |
| Kaolin | 5 % |
| Pigments | 3 % |

### *Composition cosmétique :

| *Phase huileuse :* | |
|---|---|
| Stéarate de glycéryle/stéarate de PEG-100 (Arlacel 165 | |
| vendu par la société ICI) (émulsionnant) | 2,80 |
| Alcool stéarylique | 0,75 |
| Lanoline liquide | 0,30 |
| Fraction liquide de beurre de karité | 2,00 |
| Huile d'amande d'abricot | 14,0 |

| *Phase aqueuse :* | |
|---|---|
| Glycérine | 3,00 |
| Conservateur | 0,10 |
| Eau | qsp 95 % |
| *Grains lipidiques* | 5,00 % |

Le procédé de préparation est le même que dans l'exemple 1. L'émulsionnant utilisé a une valeur de HLB de 11. On obtient un lait démaquillant qui nettoie bien la peau tout en la laissant douce et hydratée.

### EXEMPLE 5 : Lait démaquillant

### *Composition des grains lipidiques :

| | |
|---|---|
| Lipocire DM (vendu par la société Gattefosse) | 88 % |
| Monodiisostéarate de glycéryle | 5 % |
| Farine de froment | 5 % |
| Pigments | 2 % |

### *Composition cosmétique :

| *Phase huileuse :* | |
|---|---|
| Stéarate de sorbitan (émulsionnant) | 2,5 |
| Octanoate de cétéaryle | 11,0 |
| Huile de silicone volatile | 5,0 |

| *Phase aqueuse :* | |
|---|---|
| Polysorbate 60 (émulsionnant) | 2,5 |
| Carbomer | 0,5 |
| Glycérine | 5,0 |
| Eau | qsp 95 % |
| *Grains lipidiques* | 5 % |

Le procédé de préparation est le même que dans l'exemple 1. Le mélange d'émulsionnants utilisés a une valeur de HLB de 9,8. On obtient un lait démaquillant qui nettoie bien la peau tout en la laissant douce et hydratée.

## Revendications

1. Composition de nettoyage de la peau sous forme d'émulsion huile-dans-eau, caractérisée en ce qu'elle comprend un émulsionnant ayant une balance hydrophile-lipophile inférieure ou égale à 12 et des grains lipidiques solides non hydrophiles ayant un point de fusion inférieur à 50°C, ces grains incluant un actif nettoyant.

2. Composition de nettoyage selon la revendication 1, caractérisée en ce que l'actif nettoyant est choisi parmi les farines de céréale, les argiles ou les huiles démaquillantes.

3. Composition de nettoyage selon la revendication 1 ou 2, caractérisée en ce que l'émulsionnant a une balance hydrophile-lipophile allant de 6 à 12.

4. Composition de nettoyage selon l'une quelconque des revendications précédentes, caractérisée en ce que les grains sont constitués d'au moins une substance lipidique solide non hydrophile choisie dans le groupe constitué par les triglycérides d'acides gras saturés linéaires ayant de 8 à 18 atomes de carbone, les beurres ou fractions solides de corps gras végétaux et les cires de silicone.

5. Composition de nettoyage selon l'une quelconque des revendications précédentes, caractérisée en ce que les grains sont présents dans l'émulsion en une proportion allant de 0,5 % à 10 % en poids par rapport au poids total de la composition.

6. Composition de nettoyage selon l'une quelconque des revendications précédentes, caractérisée en ce que les grains ont un diamètre moyen est allant de 50 à 2.000 µm.

7. Composition de nettoyage selon l'une quelconque des revendications précédentes, caractérisée en ce que les grains sont obtenus par cryobroyage.

8. Composition de nettoyage selon l'une quelconque des revendications précédentes, caractérisée en ce que les grains contiennent au moins un adjuvant.

9. Composition de nettoyage selon l'une quelconque des revendications précédentes, caractérisée en ce que les grains contiennent au moins un actif thermosensible.

10. Composition de nettoyage selon l'une quelconque des revendications précédentes, caractérisée en ce que l'huile représente de 10 % à 40 % en poids par rapport au poids total de l'émulsion.

11. Composition de nettoyage selon l'une quelconque des revendications précédentes, caractérisée en ce que l'émulsion contient au moins un additif choisi parmi les gélifiants, les actifs hydrophiles ou lipophiles, les parfums, les conservateurs, les matières colorantes, les agents antioxydants.

12. Composition démaquillante, caractérisée en ce qu'elle contient une composition selon les revendications 1 à 11 et en ce que l'actif nettoyant est démaquillant.

13. Procédé pour nettoyer et/ou gommer la peau du corps humain et/ou du visage et/ou du cou, caractérisé en ce qu'il consiste à appliquer sur la peau la composition selon l'une quelconque des revendications 1 à 11, à masser la peau avec la composition pour éliminer les cellules mortes et éventuellement à rincer la peau.

14. Utilisation de la composition selon l'une quelconque des revendications 1 à 11, pour nettoyer et/ou gommer la peau du corps humain et/ou du visage et/ou du cou.

## Claims

1. Skin-cleansing composition in the form of an oil-in-water emulsion, characterized in that it comprises an emulsifying agent having a hydrophilic-lipophilic balance below or equal to 12 and solid, non-hydrophilic lipid grains having a melting point below 50°C, these grains incorporating a cleansing active agent.

2. Cleansing composition according to Claim 1, characterized in that the cleansing active agent is chosen from cereal flours, clays or make-up removing oils.

3. Cleansing composition according to Claim 1 or 2, characterized in that the emulsifying agent has a hydrophilic-lipophilic balance ranging from 6 to 12.

4. Cleansing composition according to any one of the preceding claims, characterized in that the grains consist of at least one solid, non-hydrophilic lipid substance chosen from the group consisting of linear saturated fatty acid triglycerides having from 8 to 18 carbon atoms, butters or solid fractions of plant fatty substances and silicone waxes.

5. Cleansing composition according to any one of the preceding claims, characterized in that the grains are present in the emulsion in a proportion ranging from 0.5% to 10% by weight relative to the total weight of the composition.

6. Cleansing composition according to any one of the preceding claims, characterized in that the grains have an average diameter ranging from 50 to 2000 µm.

7. Cleansing composition according to any one of the preceding claims, characterized in that the grains are obtained by very-low-temperature grinding.

8. Cleansing composition according to any one of the preceding claims, characterized in that the grains contain at least one adjuvant.

9. Cleansing composition according to any one of the preceding claims, characterized in that the grains contain at least one heat-sensitive active agent.

10. Cleansing composition according to any one of the preceding claims, characterized in that the oil represents from 10% to 40% by weight relative to the total weight of the emulsion.

11. Cleansing composition according to any one of the preceding claims, characterized in that the emulsion contains at least one additive chosen from gelling agents, hydrophilic or lipophilic active agents, fragrances, preserving agents, dyes and antioxidants.

12. Make-up removing composition, characterized in that it contains a composition according to Claims 1 to 11 and in that the cleansing active agent has a make-up removing action.

13. Process for cleansing and/or scrubbing the skin of the human body and/or face and/or neck, characterized in that it consists in applying to the skin the composition according to any one of Claims 1 to 11, in massaging the skin with the composition in order to remove the dead cells, and optionally in rinsing the skin.

14. Use of the composition according to any one of Claims 1 to 11, for cleansing and/or scrubbing the skin of the human body and/or face and/or neck.

## Patentansprüche

1. Zusammensetzung zur Reinigung der Haut in Form einer Öl-in-Wasser-Emulsion, dadurch gekennzeichnet, daß sie einen Emulgator mit einem HLB-(hydrophilic lipophilic balance)-Wert von höchstens 12 und nichthydrophile feste Lipidpartikel mit einem Schmelzpunkt unter 50 °C enthält, wobei diese Partikel einen reinigenden Wirkstoff enthalten.

2. Zusammensetzung zur Reinigung nach Anspruch 1, dadurch gekennzeichnet, daß der reinigende Wirkstoff unter Getreidemehlen, Tonen und Ölen zum Abschminken ausgewählt ist.

3. Zusammensetzung zur Reinigung nach Anspruch 1 oder 2, dadurch gekennzeichnet, daß der Emulgator einen HLB-(hydrophilic-lipophilic balance)-Wert im Bereich von 6 bis 12 aufweist.

4. Zusammensetzung zur Reinigung nach einem der vorhergehenden Ansprüche, dadurch gekennzeichnet, daß die Partikel aus mindestens einer festen nichthydrophilen lenstoffatomen und butterartigen oder festen Fraktionen von pflanzlichen Fetten oder Siliconwachsen ausgewählt ist.

5. Zusammensetzung zur Reinigung nach einem der vorhergehenden Ansprüche, dadurch gekennzeichnet, daß die Partikel in der Emulsion in einem Anteil im Bereich von 0,5 bis 10 Gew.-%, bezogen auf das Gesamtgewicht der Zusammensetzung, vorliegen.

6. Zusammensetzung zur Reinigung nach einem der vorhergehenden Ansprüche, dadurch gekennzeichnet, daß die Partikel einen mittleren Durchmesser im Bereich von 50 bis 2000 µm aufweisen.

7. Zusammensetzung zur Reinigung nach einem der vorhergehenden Ansprüche, dadurch gekennzeichnet, daß die Partikel durch Gefrierzerkleinerung erhalten sind.

8. Zusammensetzung zur Reinigung nach einem der vorhergehenden Ansprüche, dadurch gekennzeichnet, daß die Partikel mindestens einen Hilfsstoff enthalten.

9. Zusammensetzung zur Reinigung nach einem der vorhergehenden Ansprüche, dadurch gekennzeichnet, daß die Partikel mindestens einen wärmeempfindlichen Wirkstoff enthalten.

10. Zusammensetzung zur Reinigung nach einem der vorhergehenden Ansprüche, dadurch gekennzeichnet, daß das Öl 10 bis 40: Gew.-% des Gesamtgewichts der Emulsion ausmacht.

11. Zusammensetzung zur Reinigung nach einem der vorhergehenden Ansprüche, dadurch gekennzeichnet, daß die Emulsion mindestens einen Wirkstoff enthält, der unter Gelbildnern, lipophilen oder hydrophilen Wirkstoffen, Parfums, Konservierungsmitteln, Färbemitteln und Antioxidantien ausgewählt ist.

12. Zusammensetzung zum Abschminken, dadurch gekennzeichnet, daß sie eine Zusammensetzung nach den Ansprüchen 1 bis 11 enthält und der reinigende Wirkstoff abschminkend wirkt.

13. Verfahren zur Reinigung und/oder zum Peeling der Haut des menschlichen Körpers und/oder des Gesichts und/oder des Halses, dadurch gekennzeichnet, daß es darin besteht, eine Zusammensetzung nach einem der Ansprüche 1 bis 11 auf die Haut aufzubringen, die Haut mit der Zusammensetzung zu massieren, um die abgestorbenen Zellen zu entfernen, und ggfs. die Haut zu spülen.

14. Verwendung der Zusammensetzung nach einem der Ansprüche 1 bis 11 zur Reinigung und/oder zum Peeling der Haut des menschlichen Körpers und/oder des Gesichts und/oder des Halses.
